(19) Europäisches Patentamt
European Patent Office
Office européen des brevets

(11) **EP 3 251 681 B1**

(12) **EUROPEAN PATENT SPECIFICATION**

(45) Date of publication and mention
of the grant of the patent:
**23.12.2020   Bulletin 2020/52**

(51) Int Cl.:
*A61K 36/18* (2006.01)      *A61K 36/185* (2006.01)
*A61K 9/48* (2006.01)      *A61P 3/06* (2006.01)
*A61P 3/10* (2006.01)      *A61P 15/00* (2006.01)
*A61P 15/08* (2006.01)      *A61P 37/08* (2006.01)
*A61P 43/00* (2006.01)

(21) Application number: **16743495.0**

(22) Date of filing: **28.01.2016**

(86) International application number:
**PCT/JP2016/052548**

(87) International publication number:
**WO 2016/121892 (04.08.2016 Gazette 2016/31)**

(54) **COMPOSITION FOR AMELIORATING INFERTILITY**

ZUSAMMENSETZUNG ZUR UNFRUCHTBARKEITSLINDERUNG

COMPOSITION D'AMÉLIORATION DE L'INFERTILITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2015   JP 2015014907
23.04.2015   JP 2015088110**

(43) Date of publication of application:
**06.12.2017   Bulletin 2017/49**

(73) Proprietors:
• **Jinno Masao
Yokohama-shi, Kanagawa 225-0023 (JP)**
• **Hayashikane Sangyo Co., Ltd.
Shimonoseki-shi
Yamaguchi 750-8608 (JP)**

(72) Inventors:
• **YAMADA Michio
Shimonoseki-shi
Yamaguchi 750-8608 (JP)**
• **TAKESHITA Shoko
Shimonoseki-shi
Yamaguchi 750-8608 (JP)**
• **UEMURA Tomohiro
Shimonoseki-shi
Yamaguchi 750-8608 (JP)**
• **JINNO Masao
Kanagawa 225-0023 (JP)**

(74) Representative: **Plasseraud IP
66, rue de la Chaussée d'Antin
75440 Paris Cedex 09 (FR)**

(56) References cited:
WO-A1-2014/126199      JP-A- H11 199 500
JP-A- 2001 048 766      JP-A- 2010 077 123
JP-A- 2010 202 580      JP-A- 2014 037 399
US-B2- 7 494 671

• **Anupama Prabhakar: "Singhara (Water caltrop)
Information, Benefits, Uses and More", , 28
November 2012 (2012-11-28), XP002774318,
Retrieved from the Internet:
URL:www.bimbima.com/ayurveda/health-benefi
ts-of-singhara/1550/ [retrieved on 2017-10-02]**
• **PRAFULLA ADKAR ET AL: "Trapa bispinosa
Roxb.: A Review on Nutritional and
Pharmacological Aspects", ADVANCES IN
PHARMACOLOGICAL SCIENCES, vol. 2014, 1
January 2014 (2014-01-01), pages 1-13,
XP055411727, US ISSN: 1687-6334, DOI:
10.1155/2014/959830**
• **DATABASE EPODOC [Online] EUROPEAN
PATENT OFFICE, THE HAGUE, NL; 24 December
2008 (2008-12-24), Benshan Chen: "Medicament
for treating dropsy or emphraxis of oviduct
induced barrenness", XP002774319, Database
accession no. CN-200810058772-A & CN 101 327
306 A (BENSHAN CHEN [CN]) 24 December 2008
(2008-12-24)**

- **ADKAR P P ET AL: "Effect of hydroethanol extract of leaves of Trapa Bispinosa Roxb on Histological Assessment in Reproductive System of Albino Mice", JOURNAL OF PHARMACY RESEARCH, ASSOCIATION OF PHARMACEUTICAL INNOVATORS, INDIA, vol. 8, no. 4, 1 January 2014 (2014-01-01), pages 570-575, XP009500492, ISSN: 0974-6943**
- **YASUDA MIDORI ET AL: "Inhibitory effects of polyphenols from water chestnut (Trapa japonica) husk on glycolytic enzymes and postprandial blood glucose elevation in mice", FOOD CHEMISTRY, ELSEVIER LTD, NL, vol. 165, 23 May 2014 (2014-05-23), pages 42-49, XP029036401, ISSN: 0308-8146, DOI: 10.1016/J.FOODCHEM.2014.05.083**
- **SHOKO TAKESHITA ET AL.: 'Tobishi (Trapa bispinosa) Kahi Chushutsubutsu ni yoru Ko-Glycation Sayo' THE JAPANESE SOCIETY OF NUTRITION AND FOOD SCIENCE TAIKAI KOEN YOSHISHU vol. 65TH, 2011, page 244, XP008185821**
- **MASAO JINNO: 'Ranso Yobino Teika no Atarashii Chiryo Senryaku: To Taisha to Shumatsu Toka Sanbutsu no Juyosei' JAPAN SOCIETY OF ASSISTED REPRODUCTION ZASSHI vol. 16, no. 2, 2013, pages 58 - 60, XP008185856**
- **MASAO JINNO ET AL.: 'Shumatsu Toka Sanbutsu (AGE) ni yoru Nenrei, day-3-FSH to Dokuritsu shita Jusho Ranso Kino Shogai no Soki Shindan: Atarashii Funin Chiryoho no Kokoromi' OBSTETRICAL AND GYNECOLOGICAL PRACTICE vol. 60, no. 3, 2011, pages 469 - 476, XP008185825**
- **JINNO, M. ET AL.: 'Accumulation of advanced glycation endproducts (AGE) are novel markers to detect women with poor IVF/ICSI outcomes independently of age and day-3-FSH levels: Possible novel therapy for poor responders by decreasing AGE' JOURNAL FUR REPRODUKTIONSMEDIZIN UND ENDOKRINOLOGIE vol. 7, no. 4, 2010, pages 275 - 276, XP055402759**
- **SHINJI KOMORI ET AL.: 'Clinical Conference (Seishoku Naibunpitsu Ryoiki); 1.Funin Shinryo no Mondaiten to Taisaku 3)Immunological Infertility' ACTA OBSTETRICA ET GYNAECOLOGIA JAPONICA vol. 59, no. 9, 2007, pages N.437 - N.441, XP008185823**
- **NATSUKI UGAJIN ET AL.: 'Seisho Allergy Kanja ni Taishi IVF-ET o Okonai Kyoji o Eta Shorei' JOURNAL OF JAPAN SOCIETY FOR REPRODUCTIVE MEDICINE vol. 52, no. 4, 2007, page 328, P222, XP008185816**

**Description**

Technical Field

The invention relates to a composition for ameliorating infertility that is safe, having high activity and applicable to a variety of conditions relating to infertility.

Background Art

According to a definition by World Health Organization (WHO), "Infertility" refers to "a disease of the reproductive system defined by the failure to achieve a clinical pregnancy after 12 months or more of regular unprotected sexual intercourse". In the infertility, there are both cases caused by male and caused by female, the former is referred to as male infertility and the latter is referred to as female infertility. As causes of male infertility, for example, possible factors include:

(1) sperm production dysfunction such as azoospermia, oligozoospermia and asthenozoospermia, which is said to account for about 90% of male infertility.
(2) other factors such as varicocele, obstructive azoospermia, congenital absence of the vas deferens, pyospermia, aspermia, retrograde ejaculation, erectile dysfunction (ED), Intravaginal ejaculation disorder, etc.

As causes of female infertility, for example, possible factors include:

(1) ovulation failure: central of ovulation disorders (endocrine dysfunction), ovarian dysfunction, ovarian cysts, corpus luteum unruptured follicle, hyperprolactinemia, etc.
(2) egg tract disorders: tubal adhesions, fallopian tube stenosis, tubal occlusion, hydrosalpinx, etc.
(3) cervical disorders: cervical mucus dysfunction, formation of anti-sperm antibodies, cervical stenosis, etc.
(4) endometriosis: chocolate cysts, adenomyosis, etc.
(5) implantation failure: uterine fibroids, uterine malformations, corpus luteum dysfunction, endometrial polyps, etc.

Therapy of male infertility includes a collection of sperms from semen, vas deferens and testes and artificial insemination or microinsemination, drug therapy such as hormone replacement therapy, etc. Therapy of female infertility includes administration of, ovulation inducing agent, pharmacotherapy for ovulation disorders, cervical mucus dysfunction, hyperprolactinemia, etc., advanced reproductive medicine such as artificial insemination, in vitro fertilization, etc. As the ovulation-inducing agent, oral medicines such as clomiphene (for example, see Patent Document 1), cyclofenil, injection agents such as gonadotropin are widely used. For the pharmacotherapy for hyperprolactinemia, dopamine agonists such as bromocriptine, terguride, cabergoline have been used. Also, improvement in the pregnancy rate of the patients of polycystic ovarian syndrome and non-polycystic ovary syndrome by administration of metformin, an insulin sensitizing agents has been reported (see Non-Patent Document 1).
An ayurvedic properties and action of shelled fruit and dried kernel flour of *Trapa natans* (Singhara), for example, improvement of fertility in women, conception and chances in healthy implantation of embryo, decrease of risk of threatened abortion and cure of menorrhagia are described in Non Patent Document 2. A review on nutritional and pharmacological aspects of *Trapa bispinosa Roxb.* in ethnopharmacological use is described in Non Patent Document 3. Medicaments for remedying infertility caused by oviduct edema or tubal occlusion made from weak water chestnut is described in Patent Document 2.

Prior Art Document

Patent Document

Patent Document 1: JPA 2009-503096
Patent Document 2: CN 101327306 A

Non Patent Document

Non Patent Document 1: Masao Jinno, Kenichi Kondou, Koji Teruya, "Low-dose metformin improves pregnancy rate in in vitro fertilization repeaters without polycystic ovary syndrome: Prediction of effectiveness by multiple parameters related to insulin resistance", HORMONES 2010, 9(2): p. 161-170.
Non Patent Document 2: Anupama Prabhaker, "Singhara (Water caltrop) Information, Benefits, Used and More", 28 November 2012 (2012-11-28), URL: www.bimbiba.com/ayurveda/health-benefits-of-singhara/1550/
Non Patent Document 3: Prafulla Adkar et al.: "Trapa bispinosa Roxb.: A Review on Nutritional and Pharmacological Aspects", Advances in Pharmacological Scieneces, Vol. 2014, 1 January 2014, pages. 1-13.

SUMMARY

The invention is as defined in the claims.

Problem to be Solved by the Invention

However, the medicament used in the pharmacotherapy of infertility has a variety of side effects including those could be life-threating such as multiple pregnancy and ovarian hyperstimulation syndrome (For example, see Takumi Yauchihara, " Reproductive Medicine and Bioethics: Multiple Pregnancy and its Problems ", Trends in the Science, Japan Science Council, April 1999, p. 31-37.). In addition to the factors described above, it may be considered that symptoms due to lifestyle-related diseases and the like such as the glycation stress allergic diseases involve in infertility in a complex manner (for example, see, Shinji Komori, "Clinical Conference (Reproductive Endocrine area) 1. Problems and Measures of Infertility Clinics; 3) Immunity infertility" ACTA OBSTERICA ET GYNAECOLOGICA JAPONICA (Japan Society of Obstetrics and Gynecology), Vol. 59, No. 9). However, effective treatment for such cases has not been established so far. When a plurality of medicaments is administered simultaneously, depending on the combination, the risk of unexpected serious side effects and the like may exist. Under these circumstances, establishment of safe and effective medicaments and therapy for infertility in which a variety of factors involve in a complex manner.
The object of the invention is to provide a composition for ameliorating infertility that is safe, having high activity and applicable to a variety of conditions relating to infertility while taking such kinds of hitherto known problems into consideration.

Means for Solving Problem

The invention that suits the aforementioned object is to solve the problem mentioned above by providing a composition for use in ameliorating infertility comprising one or more compounds contained in the pericarp of a plant of the genus Trapa as an active ingredient.
The composition for use in ameliorating infertility according to the invention comprises an extract of the pericarp as the active ingredient.
The composition for use in ameliorating infertility according to the invention preferably has an inhibition activity against one or more reactions involving a formation of advanced glycation end products from proteins and saccharides.
The composition for use in ameliorating infertility according to the invention preferably has an enhancing activity against one or more reactions involving a decomposition of the advanced glycation end products.
The composition for use in ameliorating infertility according to the invention preferably has an activity to reduce allergic symptoms.

Effect of the Invention

The active ingredient of the composition for use in ameliorating infertility according to the invention is one or more compounds contained in pericarp of a plant of the genus Trapa having meal experiences. The composition for use in ameliorating infertility according to the invention has high activity for ameliorating infertility as well as its safety has been confirmed. Therefore, the invention provides the composition for use in ameliorating infertility that is safe, having high activity.

Brief Description of Drawings

Fig. 1    is a graph showing the result of $\alpha$-dicarbonyl bond cleavage activity measurement.
Fig.2    is a graph showing the result of AGE crosslink cleavage activity measurement.

Embodiment of the Invention

In the following, embodiments of the invention will be explained with reference to drawings for better understanding the invention.

The composition for use in ameliorating infertility according to one embodiment of the invention (hereinafter abbreviated to "the composition for use in ameliorating infertility " or simply "the composition") comprises one or more compounds contained in the pericarp of a plant of the genus Trapa as an active ingredient.

As one or more compounds contained in the pericarp of a plant of the genus Trapa, an active ingredient, for example, the composition for oral administration may contain pulverized or finely powdered pericarp and for the production of the composition for injection, an extract of pericarp is preferably used.

The one or more compounds contained in the pericarp of a plant of the genus Trapa is an extract of the pericarp. Hereinafter a method for producing the extract of the one or both of the pericarp and the fruit will be illustrated.

The pericarp of the plant of the genus Trapa may be any one of fresh fruit, the pericarp took from the fresh fruit, the pericarp dried after taking and the pericarp took from dried fruit. To improve extract efficiency, pretreatment such as crushing or grinding may be conducted by any method prior to solvent extraction.

The plants of genus Trapa used for the extraction is not particularly limited and examples include *Trapa japonica, Trapa natans L. ver. japonica, Trapa incisa* and *Trapa bispinosa Roxb.*

As a solvent for extraction, water, aqueous solution, a solvent mixture of water and any one more solvents miscible with water in any ratio (aqueous solvent) and a preferred solvent is water, methanol, ethanol and an aqueous solvent of any two or more mixed in any ratio, and particularly preferred solvent is the aqueous solvent of water and ethanol, an organic solvent approved as a food additive mixed in any ratio. Temperature of the extraction solvent may be any temperature above room temperature and the boiling point of the extraction solvent or less, which is preferably determined taking extraction efficiency, heat resistance and volatility of the substance to be extract and the like into consideration.

When water or aqueous solvent is used as the extract solvent, it may optionally contain acid, base and salt and the like so as to enhance extraction efficiency. Temperature and pH of water used for the extraction is not particularly limited, however, with respect to pH, taking the use to a living organism into consideration, around neutral pH, particularly pH of 4 to 9 is preferred and pH of 6 to 8 is more preferred. Optionally, hot extraction solvent may be used to improve the extraction efficiency.

Hot water extraction may be carried out by any method known in the art such as, for example, by a method in which one or both of the pericarp and the fruit of the plant of the genus Trapa is mixed in the solvent for predetermined time and solid is separated by filtration, centrifugation, decantation and the like, by a continuous extraction method such as Soxhlet extraction.

Prior to separating one or more compounds from the solvent extract of the pericarp of the plant of the genus Trapa, pretreatment such as dialysis, ultrafiltration, filtration and column chromatography may be carried out to remove high molecular weight components and insoluble matter and the like.

When the insoluble matter is removed by filtration, absorbent such as active charcoal, bentonite and Celite (trademark) and filter aid may be optionally added to remove impurity. Particularly, when the composition is used as the liquid extract, sterile filtration by a membrane filter and the like is preferably carried out.

The separation of the hot water extract which is optionally pretreated as described above may be carried out by any method known in the art such as column chromatography, reverse phase chromatography, ion chromatography to fractionate a fraction having high activity concerning to amelioration of infertility as described below.

The activity concerning to amelioration of infertility includes inhibition activity against one or more reactions involving a formation of advanced glycation end products from proteins and saccharides. Advanced glycation end products (AGEs), which is also referred to as glycated proteins or Mailard reaction products, are protein derivatives having various structures form by non-enzymatic reaction of reducing saccharide such as glucose and amino group of protein. The AGEs involve the onset and progression of a variety of diseases as a result of deterioration of extracellular matrix proteins, membrane proteins and intracellular proteins thorough the glycation of these proteins and deterioration of cellular function relying on these proteins or as a result of cellular response caused by receptors for the AGEs.

For example, the AGEs are recognized by RAGE, an AGEs receptor, production of intracellular oxidative stress substance is enhanced by intracellular NADPE oxidase, which modulates the expression in epithelium cells, which is considered to be a cause of various diabetic vascular disorders.

Recently, it is also suggested that the AGEs involve cardiovascular disorders such as myocardial infarction and atherosclerosis, Alzheimer's disease, Parkinson's disease, neurodegenerative diseases such as amyotrophic lateral sclerosis, brain damage and liver failure due to alcoholism, diabetic nephropathy, diabetic retinopathy, diabetic complications such as diabetic neuropathy, abnormal bone metabolism such as osteoporosis, aging, insulin resistance, tumor growth and metastasis and the like, in addition to the diabetic vascular disorders.

Diabetes is known to be one of the causes of infertility and menstrual irregularity. Although not necessarily clear, its mechanism is thought to be the inhibition of the function of insulin, which also plays an important role in ovulation and damage of ovaries, fallopian tubes, cervix, endometrium caused by the AGEs. In addition, pregnancy diabetes is a risk factor of various disorders such as placental abruption, premature birth and stillbirth, abnormal growth of the fetus due to excessive supply of saccharides and hypoglycemia after birth.

Among the reactions affording the AGEs, the reaction to be inhibited by the composition for ameliorating infertility may be any one or more of formation of Schiff base thorough the reaction of amino group of proteins and reducing saccharides, formation of 1,2-enaminol or 2,3-enediol by Amadori rearrangement, decomposition of Amadori rearrangement product and formation of polymerization product of the decomposition product and amino acid, peptide or protein.

The inhibition activity against one or more reactions involving a formation of advanced glycation end products from proteins and saccharides may be evaluated by, for example, measuring the AGEs formed by reacting serum protein such as human serum albumin and glucose (for example at 60°C for 40 hours) with fluorescence spectroscopy and estimating inhibitory ratio of formation (%) in the presence of the composition for ameliorating infertility or by evaluating the influence of the administration of the composition for ameliorating infertility on the concentration of AGEs (such as HbAlc and pentosidine) in blood and the like. The mechanism of the inhibition of one or more reactions involving a formation of the AGEs by one or more compounds contained in one or both of pericarp and fruit of a plant of the genus Trapa and the relationship between such inhibition activity and amelioration of infertility is not necessarily certain, however, the composition for ameliorating infertility comprising such compounds is shown to ameliorate infertility as well as suppress the formation of the AGEs and decompose the AGEs.

Another activity which may involve ameliorating infertility includes an enhancing activity against one or more reactions involving a decomposition of the advanced glycation end products (AGEs). The reaction involving the decomposition of AGEs include cleavage reactions formed by formation of Schiff base thorough the reaction of amino group of proteins and reducing saccharides, formation of 1,2-enaminol or 2,3-enediol by Amadori rearrangement, decomposition of Amadori rearrangement product and formation of polymerization product of the decomposition product and amino acid, peptide or protein and an example of the reactions used for evaluating the enhancing activity against one or more reactions involving a decomposition of the AGEs includes a cleavage reaction of $\alpha$-dicarbonyl bond, which is characteristic of the AGEs, and a cleavage reaction of a crosslink formed by the reaction between proteins such as collagen and the AGEs.

Another activity which may involve ameliorating infertility includes an activity to reduce allergic symptoms. Allergic diseases such as atopic dermatitis, allergic rhinitis and pollen allergies pointed out to be associated with endometriosis and it is also said to be a cause of infertility caused by the immune system. The mechanism of the reduction of allergic symptoms by one or more compounds contained in one or both of pericarp and fruit of a plant of the genus Trapa and the relationship between such inhibition activity and amelioration of infertility is not necessarily certain, however, the composition for ameliorating infertility comprising such compounds is shown to ameliorate infertility as well as reducing the allergic symptoms.

By mixing the composition for use in ameliorating infertility with carriers and the like, the composition may be used as a medical composition having one or both of therapeutic effect and preventing effect against infertility and associated diseases and conditions. Administration form of the medical composition to human or animals includes oral, rectal, parenteral (for example, intravenous, intramuscular, subcutaneous, etc.). Although the dosage is to be adjusted appropriately depending on dosage forms of the pharmaceutical compositions, method of administration, purpose of administration and the age, weight and condition of the administration subject and it is difficult to determine unambiguously, in humans, generally the amount of active ingredient contained in the formulation is preferably 1 day 0.1 to 2000mg/day adult human. Naturally, the dosage can vary depending on various factors and in some cases smaller dose than the dosage as mentioned above may be sufficient, or in other case the dosage exceeding the range as mentioned above may be required.

When the composition is formulated in an oral dosage form, it may be formulated in the form of tablets, granules, powders, capsules, coatings, solutions, suspension and the like, and when the composition is formulated in parenteral dosage form, it may be formulated in the form of injections, infusions, suppositories and the like. Any known method may be used for formulation. For example, the composition for ameliorating infertility may be admixed with pharmaceutically acceptable carrier or diluent, stabilizers and other desired additives to formulate in the desired dosage form as described above.

Food products containing the composition for use in ameliorating infertility may be foods to which the composition for use in ameliorating infertility is added or may be in the form of capsules, tablets or in any form usually used for food or supplements. The kind of food to which the composition is to be added is not particularly limited and it may be added to, for example, staple food, side dish, confectionery and seasoning including: liquid (fluid) foods such as coffee, juice, refreshing drink, beer, milk, Japanese soy bean soup (Miso-soup), soup, black tea, green tea, nutrition, syrup, margarine and jam; solid food such as cocked rice, bread, potato products, rice cake, candy, chocolate, dried seasoning powder (Furikake), ham, sausage and candy. The food product for certain application may be formulated in the form of powders, granules, tablets and the like. Also, the composition optionally may be mixed with excipients, fillers, binders, thickeners, emulsifiers, colorants, flavors, food additives, flavorings and the like.

In addition to food and supplements provided for human consumption, for administering the composition for ameliorating infertility to female mammals such as live stocks or pets, it may be mixed mixing in advance in the feed of the raw material on, it can be prepared as functional feed. It is also possible to administer the composition for ameliorating infertility by adding to the feed. In other words, the foods containing the composition for ameliorating infertility as an active ingredient may be used as functional feed that is safe and has an activity of improving infertility of livestock such as pigs, cows, horses and sheep, etc., and pets (dogs, cats) by adding to the feed for these animals.

EXAMPLES

Next, examples carried out to confirm the effect of the invention will be illustrated. Example 1: Preparation of an extract of pericarp of Trapa and an oral formulation containing the same

(1) Preparation of hot water extract of plant of the genus Trapa (hereinafter abbreviated to "Trapa pericarp extract")

Fruit of harvested *Trapa bispinosa* was dried and dried pericarp was collected. The dried pericarp was pulverized by a food processor, extracted with hot water (6 weight part of hot water of 90°C was used for 1 weight part of the dried pericarp) and the extract was concentrated in predetermined concentration rate so that polyphenol content of resultant Trapa pericarp extract is 25 weight% or more. 33 weight% of dextrin is added to 67 weight% of the concentrate and the mixture was spray dried using a spray drier. The powder thus obtained (polyphenol content of 25 weight% or more) is used in tests described below as a Trapa pericarp extract.

(2) Preparation of oral formulation containing the Trapa pericarp extract

To 100 mg of the Trapa pericarp extract prepared in (1) as described above, 187 mg of corn starch and 3 mg of calcium stearate as excipients, which was enclosed in hard gelatin capsule. In Examples 2 to 4 to be described later, subjects were asked to take one hard capsules a day before meals with water or tepid water. The formulation prepared using dextrin instead of the Trapa pericarp extract (indistinguishable on the oral dosage including the Trapa pericarp extract in appearance) was used as a placebo.

Example 2_: Influence of continuous administration Trapa pericarp extract on blood saccharide level, blood AGEs level and blood cholesterol level

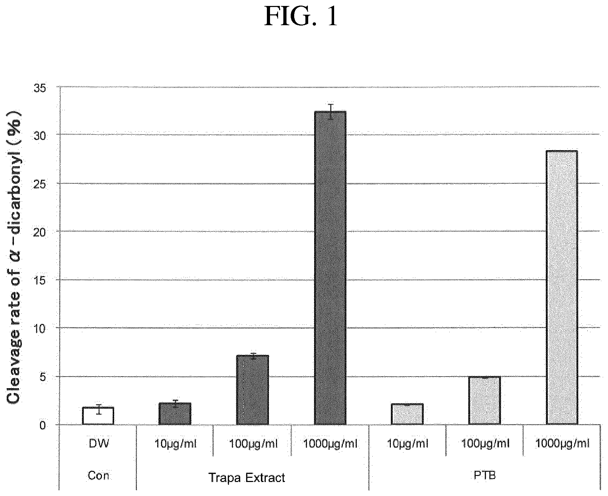

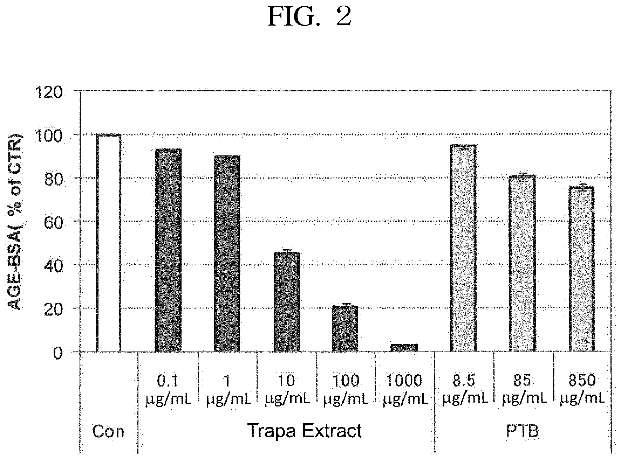

(1) 50 female subjects were divided in Trapa pericarp extract administered group and placebo administered group (25 for each) and the oral formulation containing the Trapa pericarp extract (indicated "Trapa extract" in Tables 1 to 6, Fig. 1 and 2) for the former and the placebo for the latter were administered for 12 weeks according to double blind protocol in the dosage as described above, in Example 1 (2). Measurement of blood test (blood HbAlc level, blood neutral lipid level, blood cholesterol level (total cholesterol level, HDL-cholesterol level and LDL-cholesterol level) and blood pentosidin level) was carried out. Results of each of the measurements are shown in Table 1 to 6. In the Tables, "0W", "4W", "8W" and "12W" refer to the result of week 0, week 4, week 8 and week 12, respectively, "Mean" refers to average value, "SD" refers to standard deviation, "SE" refers to standard error and "Wilcoxon" refers to the result of Wilcoxon signed-rank test, respectively.

Table 1

|  |  | HbAlc (%) | | | |
|---|---|---|---|---|---|
|  |  | 0W | 4W | 8W | 12W |
| Trapa Extract | Mean | 5.06 | 5.06 | 5.06 | 4.98 |
|  | SD | 0.24 | 0.23 | 0.25 | 0.23 |
|  | SE | 0.05 | 0.05 | 0.05 | 0.05 |
|  | Mean±SE | 5.06±0.05 | 5.06±0.05 | 5.06±0.05 | 4.98±0.05 |
|  | Wilcoxon (vs. 0W) |  | 0.79 | 0.70 | 0.01 * |

(continued)

| | | HbAlc (%) | | | |
|---|---|---|---|---|---|
| | | 0W | 4W | 8W | 12W |
| Placebo | Mean | 5.06 | 5.09 | 5.11 | 5.02 |
| | SD | 0.28 | 0.28 | 0.28 | 0.28 |
| | SE | 0.06 | 0.06 | 0.06 | 0.06 |
| | Mean±SE | 5.06±0.06 | 5.09±0.06 | 5.11±0.06 | 5.02±0.06 |
| | Wilcoxon (vs. 0W) | | 0.205 | 0.168 | 0.337 |

Table 2

| | | neutral lipid (mg/dL) | | | |
|---|---|---|---|---|---|
| | | 0W | 4W | 8W | 12W |
| Trapa Extract | Mean | 62.76 | 62.64 | 58.48 | 65.68 |
| | SD | 28.68 | 30.53 | 20.98 | 42.83 |
| | SE | 5.74 | 6.11 | 4.20 | 8.57 |
| | Mean±SE | 62.8±5.7 | 62.6±6.1 | 58.5±4.2 | 65.7±8.6 |
| | Wilcoxon (vs. 0W) | | 0.93 | 0.64 | 0.90 |
| Placebo | Mean | 84.84 | 67.88 | 80.24 | 80.44 |
| | SD | 74.07 | 46.84 | 65.86 | 54.44 |
| | SE | 14.81 | 9.37 | 13.17 | 10.89 |
| | Mean±SE | 84.8±14.8 | 67.9±9 .4 | 80.2±13.2 | 80.4±10.9 |
| | Wilcoxon (vs. 0W) | | 0.18 | 0.75 | 0.85 |

Table 3

| | | Total Cholesterol (mg/dL) | | | |
|---|---|---|---|---|---|
| | | 0W | 4W | 8W | 12W |
| Trapa Extract | Mean | 190.04 | 191.40 | 187.40 | 181.64 |
| | SD | 39.31 | 39.00 | 41.22 | 35.02 |
| | SE | 7.86 | 7.80 | 8.24 | 7.00 |
| | Mean±SE | 190±7.9 | 191.4±7.8 | 187.4±8.2 | 181.6±7 |
| | Wilcoxon (vs. 0W) | | 0.77 | 0.55 | 0.03 * |
| Placebo | Mean | 195.64 | 196.72 | 201.60 | 192.36 |
| | SD | 41.78 | 33.54 | 38.74 | 30.57 |
| | SE | 8.36 | 6.71 | 7.75 | 6.11 |
| | Mean±SE | 195.6±8.4 | 196.7±6.7 | 201.6±7.7 | 192.4±6.1 |
| | Wilcoxon (vs. 0W) | | 0.383 | 0.035 | 0.545 |

Table 4

|  |  | HDL-Cholesterol (mg/dL) | | | |
|---|---|---|---|---|---|
|  |  | 0W | 4W | 8W | 12W |
| Trapa Extract | Mean | 65.32 | 66.04 | 64.88 | 63.84 |
|  | SD | 12.92 | 12.71 | 11.00 | 12.31 |
|  | SE | 2.58 | 2.54 | 2.20 | 2.46 |
|  | Mean±SE | 65.3±2.6 | 66±2.5 | 64.9±2.2 | 63.8±2.5 |
|  | Wilcoxon (vs. 0W) |  | 0.252 | 0.638 | 0.222 |
| Placebo | Mean | 64.56 | 65.80 | 66.88 | 63.20 |
|  | SD | 13.42 | 13.34 | 14.13 | 11.19 |
|  | SE | 2.68 | 2.67 | 2.83 | 2.24 |
|  | Mean±SE | 64.6±2.7 | 65.8±2.7 | 66.9±2.8 | 63.2±2.2 |
|  | Wilcoxon (vs. 0W) |  | 0.627 | 0.101 | 0.115 |

Table 5

|  |  | LDL-Cholesterol (mg/dL) | | | |
|---|---|---|---|---|---|
|  |  | 0W | 4W | 8W | 12W |
| Trapa Extract | Mean | 106.88 | 108.68 | 106.00 | 100.24 |
|  | SD | 31.16 | 32.97 | 35.15 | 32.07 |
|  | SE | 6.23 | 6.59 | 7.03 | 6.41 |
|  | Mean±SE | 106.9±6.2 | 108.7±6.6 | 106±7 | 100.2±6.4 |
|  | Wilcoxon (vs. 0W) |  | 0.56 | 0.66 | 0.02 * |
| Placebo | Mean | 105.48 | 109.64 | 111.08 | 106.76 |
|  | SD | 31.42 | 29.34 | 34.48 | 29.38 |
|  | SE | 6.28 | 5.87 | 6.90 | 5.88 |
|  | Mean±SE | 105.5±6.3 | 109.6±5.9 | 111.1±6.9 | 106.8±5.9 |
|  | Wilcoxon (vs. 0W) |  | 0.135 | 0.014 * | 0.275 |

Table 6

|  |  | Blood Pentosidine Level ($\mu$g/mL) | | | |
|---|---|---|---|---|---|
|  |  | 0W | 4W | 8W | 12W |
| Trapa Extract | Mean | 0.025 | 0.002 | 0.037 | 0.026 |
|  | SD | 0.009 | 0.002 | 0.015 | 0.011 |
|  | SE | 0.0018 | 0.0004 | 0.0030 | 0.0023 |
|  | Mean±SE | 0.025±0.0018 | 0.002±0.0004 | 0.037±0.0030 | 0.026±0.0023 |
|  | Wilcoxon (vs. 0W) |  | *** | <0.001 *** | 0.706 |

(continued)

| | | Blood Pentosidine Level ($\mu$g/mL) | | | |
|---|---|---|---|---|---|
| | | 0W | 4W | 8W | 12W |
| Placebo | Mean | 0.016 | 0.012 | 0.036 | 0.014 |
| | SD | 0.006 | 0.008 | 0.012 | 0.011 |
| | SE | 0.0012 | 0.0016 | 0.0025 | 0.0022 |
| | Mean$\pm$SE | 0.016$\pm$0.0012 | 0.012$\pm$0.0016 | 0.036$\pm$0.0025 | 0.014$\pm$0.0022 |
| | Wilcoxon (vs. 0W) | | 0.008 ** | <0.001 *** | 0.050 * |

From the results shown in Table 1 to 6, significant decreasing of blood HbAlc, total blood cholesterol level, blood LDL-cholesterol level and blood pentosidine level due to the administration of the oral formulation containing Trapa pericarp extraction were observed.

Example 3: Influence of administration Trapa pericarp extract on postprandial blood saccharide level.

Four subjects with fasting blood saccharide level of 70 to 110 mg/dL (average age of 28.7 years old, 1 male and 3 female) were tested according to the protocol as described below.

Fasting blood saccharide level of the subjects who fasted 21 o'clock of the day before the test (prohibited food and drink other than water) was measure at 8 o'clock of the day of the test, after which administered oral formulation containing Trapa pericarp extract or placebo (according to double-blind detection method). After 5 minutes, subjects were fed with 200g of cocked rice and 2.5 g of dried seasoning powder (total calorie 308 kcal, carbohydrate amount of 70.2 g) as a glycemic load diet and the blood saccharide levels were measured at the time of 15 minutes, 30 minutes, 45 minutes, 90 minutes and 120 minutes after feeding. After a 1 week of wash-out period, second test was carried out according to the protocol similar to that of the first test except that the placebo were administered to the subjects to whom the oral formulation containing the Trapa pericarp extract had been administered at the first test and the oral formulation containing the Trapa pericarp extract were administered to the subjects to whom the placebo had been administered at the first test. Test results are shown in Table 7.

Table 7

| Postprandial blood saccharide level (measured value) | | |
|---|---|---|
| Time after feeding glycemic load diet | Placebo | Trapa Extract |
| 0 minute | 84.1$\pm$4.3 | 85.6$\pm$2.5 |
| 15 minute | 133$\pm$4.1 | 114.9$\pm$2.9 * |
| 30 minute | 137$\pm$9.4 | 128.3$\pm$7.4 |
| 45 minute | 115.4$\pm$5.5 | 114.8$\pm$7.7 |
| 60 minute | 102.1$\pm$4.6 | 99.4 $\pm$8 |
| 90 minute | 104.6$\pm$2.7 | 90.8$\pm$6.7 |
| 120 minute | 91.8$\pm$5.9 | 91.5$\pm$3.1 |
| mean value$\pm$standard deviation paired t-test vs. placebo *: $p<0.05$ | | |

Apparent from the result shown in Table 7, it was shown that elevation of the blood saccharide level 15 minutes after feeding is significantly suppressed by the administration of the oral formulation containing the Trapa pericarp extract.

Example 4: Measurement of $\alpha$-dicarbonyl bond cleavage activity

Cleavage activity of dicarbonyl bond was estimated by measuring a concentration of benzoic acid obtained by a decomposition of 1-phenyl-1,2-propanedione (PPD), a dicarbonyl compound using HPLC method. 4.2 mL of 98% PPD solution was dissolved in 25mL of 50 mM phosphate buffer solution (pH 7.4)/50% methanol (methanol buffer) to prepare a PPD solution so that a concentration in final reaction solution is 1.0 mM. To 900 μL of the PPD solution prepared as above, 100 μL of the solution of the Trapa pericarp extract of any concentration is added and allowed to react for 4 hours while shaking at 37°C. The reaction was quenched by adding 200 mL of 2N HCl, then the mixture was filtrated by 0.45 μm filter and used as the HPLC sample.

TSKgel ODS-80T, 150×6.0 mm (I.D.) (TOSOH Corporation) was used as an analyzation column. 50 mM phosphate buffer (pH 2.2) was used as an eluent, flow rate was 1.0 mL/min and detection wavelength was 230 nm.

Concentration of benzoic acid in each measured sample was determined using a calibration curve prepared using each predetermined concentration of benzoic acid (Wako Pure Chemical Industries) and the cleavage activity of dicarbonyl bond in the PPD structure assuming that 1.0 mM of benzoic acid was formed from 1.0 mM of PPD.

$$\text{Dicarbonyl Cleavage Activity} = 100 \times \text{Concentration of Benzoic Acid (mM)/1 mM}$$

Results are shown in Fig. 1. In Fig. 1, "Con" refers to a control and "PTB" refers to a result of a measurement using N-phenacyl thiazorium bromide (PTB) as a positive control under similar condition. Fig. 1 clearly shows that the Trapa pericarp extract exhibits the α-dicarbonyl bond cleavage activity higher than that of PTB.

Example 5: Measurement of AGE cross-link cleavage activity

AGE cross-link cleavage activity was evaluated by measuring a cross-link formed between type I-collagen and AGE-BSA using ELISA to estimate the cross-link cleavage rate associated with the addition of the Trapa pericarp extract.

To BD BioCoat Collagen I 96 well micro test plate, AGE-BSA 10 μg/mL solution (MBL) (100 μL) was added and incubated at 37°C for 4 hours. The wells were washed with 0.05% Tween 20/PBS (-) 5 times, 100 μL of samples of different concentrations dissolved in PBS (-) were added and reacted at 37°C for 20 hours. After the reaction, each well was washed with 0.05% Tween 20/PBS (-) 3 times, 100 μL of 1 μg/mL anti-bovine serum albumin (BSA) rabbit polychronal antibody solution (ROCKLAND) was added to each well as a primary antibody and incubated at room temperature for 30 minutes. After the incubation, each well was washed with 0.05% Tween 20/PBS (-) 5 times and 100 μL of 1 μg/mL HRP labeled anti- rabbit IgG antibody solution (Funakoshi) was added to each well as a primary antibody and incubated at room temperature for 30 minutes. Each well was washed with 0.05% Tween 20/PBS (-) 3 times and 100 μL of TMB Microwell Peroxidase Substrate (KPL) was added to each well after washing and reacted at room temperature for 15 minutes. The reaction was terminated by adding 100 μL of IN $H_2SO_4$ to each well and absorbance at 450 nm was measured using a microplate reader. Using measured values of sample addition group and non-sample addition group (control), remaining rate of AGE-BSA cross-link was estimated using the formula shown below.

$$\text{Remaining Rate of AGE-BSA (\% of CTR)} = 100 \times \text{Absorbance of Sample Addtion Group/Absorbance of Control}$$

The results are shown in Fig. 2. In Fig. 2, "Con" refers to a control and "PTB" refers to a result of a measurement using N-phenacyl thiazorium bromide (PTB) as a positive control under similar condition. Fig. 2 clearly shows that the Trapa pericarp extract exhibits the AGE cross-link cleavage activity higher than that of PTB.

Example 6: Test Administration to Intractable Infertility Patients (1)

As subjects, 5 female patients with intractable infertility whose repeated ART (advanced assisted reproductive technologies) resulted in unsuccessful received the administration of the oral formulation containing the Trapa pericarp extract for 3 months. After the administration of 3 months, blood HbAlc levels of every subjects decreased by 0.1 to 0.4 points. In addition, condition of atopic dermatitis of 1 subject improved, condition of allergic rhinitis (pollen allergy) of 1 subject improved and 1 subject reported that cosmetics spread on her face more easily. Among the subjects, 1 female at the age of 40s suffering from atopic dermatitis and diabetes and 13 times of external fertilization had been unsuccessful succeeded in becoming pregnant by external fertilization during the test period.

Example 7: Test Administration to Intractable Infertility Patients (2)

From July 2014 to March 2015, to 32 female subjects with intractable infertility whose repeated ART (advanced assisted reproductive technologies) resulted in unsuccessful (including 5 subjects of Example 6), under informed consent, ART was conducted while receiving the continuous administration of the Trapa pericarp extract (100 mg, once a day just before breakfast) since 1 or 2 month before the ART.

Among the total 32 patients, embryonic development improved in 24 patients (75%) in comparison with the previous ART, unchanged in 5 patients (16%) and failed in 3 patients (9%). As a result, 7 patients became a clinical pregnancy and 4 patients became a chemical pregnancy (total pregnancy rate of 34%, a clinical pregnancy rate of 22%).

About the influence of the administration of the Trapa pericarp extract on blood CML (carboxymethyl lysine: a kind of AGEs), although a tendency to decrease was observed, it is not significant (a drop from $1.3\pm0.2$ $\mu$g/mL to $1.0\pm0.2$ $\mu$g/mL; p=0.30, n=15, paired t-test). However, the analysis carried out by dividing the subject to a group of subjects of higher blood CML level (>1.0 $\mu$g/mL) prior to the administration of the Trapa pericarp extract and a group of the subject of lower blood CML level ($\leq$1.0 $\mu$g/mL) showed that significant drop of blood CML level from $2.0\pm0.2$ prior to the administration to $1.1\pm0.3$ after the administration was observed in the higher CML group (7 subjects) (p<0.01), while a tendency to increase from $0.6\pm0.05$ prior the administration to $1.0\pm0.2$ was observed in the lower CML group (8 subjects) (p=0.09).

The clinical pregnancy rate of 43% (3 of 7 subjects) of the higher CML group and 0% (0 of 8 subjects) of the lower CML group shows a tendency of higher clinical pregnancy rate of the higher CML group (p=0.08, Fisher's exact test). These results suggest that the administration of the Trapa pericarp extract is effective for the subject of higher blood AGEs level and the activity to drop blood AGEs level is attributable to the increasing in the pregnancy rate.

**Claims**

1. A composition for use in ameliorating infertility comprising, as the active ingredient, an extract of the pericarp of a plant of the genus Trapa.

2. The composition for use in ameliorating infertility according to Claim 1, wherein the composition has an inhibition activity against one or more reactions involving a formation of advanced glycation end products from proteins and saccharides.

3. The composition for use in ameliorating infertility according to any one of Claim 1 or 2, wherein the composition has an enhancing activity against one or more reactions involving a decomposition of the advanced glycation end products.

4. The composition for use in ameliorating infertility according to any one of Claims 1 to 3, wherein the composition has an activity to reduce allergic symptoms.

**Patentansprüche**

1. Zusammensetzung zur Verwendung bei der Linderung von Unfruchtbarkeit, umfassend als aktiven Bestandteil einen Extrakt aus der Fruchtwand einer Pflanze der Gattung Trapa.

2. die Zusammensetzung zur Verwendung bei der Verbesserung der Unfruchtbarkeit nach Anspruch 1, wobei die Zusammensetzung eine Hemmwirkung gegen eine oder mehrere Reaktionen aufweist, die eine Bildung von fortgeschrittenen Glykierungsendprodukten aus Proteinen und Sacchariden beinhalten.

3. die Zusammensetzung zur Verwendung bei der Verbesserung der Unfruchtbarkeit nach einem der Ansprüche 1 oder 2, wobei die Zusammensetzung eine verstärkende Aktivität gegen eine oder mehrere Reaktionen aufweist, die eine Zersetzung der fortgeschrittenen Glykierungsendprodukte beinhalten.

4. die Zusammensetzung zur Verwendung bei der Linderung von Unfruchtbarkeit nach einem der Ansprüche 1 bis 3, wobei die Zusammensetzung eine Aktivität zur Verminderung allergischer Symptome aufweist.

**Revendications**

1. Composition à utiliser pour améliorer l'infertilité comprenant, en tant que principe actif, un extrait du péricarpe d'une plante du genre Trapa.

2. Composition à utiliser pour améliorer l'infertilité selon la revendication 1, dans laquelle la composition a une activité d'inhibition contre une ou plusieurs réactions impliquant une formation de produits terminaux avancés de glycation à partir de protéines et de saccharides.

3. Composition à utiliser pour améliorer l'infertilité selon l'une quelconque de la revendication 1 ou 2, dans laquelle la composition a une activité facilitante contre une ou plusieurs réactions impliquant une décomposition des produits terminaux avancés de glycation.

4. Composition à utiliser pour améliorer l'infertilité selon l'une quelconque des revendications 1 à 3, dans laquelle la composition a une activité pour réduire des symptômes allergiques.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009503096 A

- CN 101327306 A

**Non-patent literature cited in the description**

- **MASAO JINNO ; KENICHI KONDOU ; KOJI TERUYA.** Low-dose metformin improves pregnancy rate in in vitro fertilization repeaters without polycystic ovary syndrome: Prediction of effectiveness by multiple parameters related to insulin resistance. *HORMONES,* 2010, vol. 9 (2), 161-170
- **ANUPAMA PRABHAKER.** *Singhara (Water caltrop) Information, Benefits, Used and More,* 28 November 2012, www.bimbiba.com/ayurveda/health-benefits-of-singhara/1550

- **PRAFULLA ADKAR et al.** Trapa bispinosa Roxb.: A Review on Nutritional and Pharmacological Aspects. *Advances in Pharmacological Scieneces,* 01 January 2014, vol. 2014, 1-13
- **TAKUMI YAUCHIHARA.** Reproductive Medicine and Bioethics: Multiple Pregnancy and its Problems. *Trends in the Science, Japan Science Council,* April 1999, 31-37
- Clinical Conference (Reproductive Endocrine area) 1. Problems and Measures of Infertility Clinics; 3) Immunity infertility. **SHINJI KOMORI.** ACTA OBSTERICA ET GYNAECOLOGICA JAPONICA. Japan Society of Obstetrics and Gynecology, vol. 59